# EUROPEAN PATENT SPECIFICATION

(11) **EP 2 249 860 B1**
(45) Date of publication and mention of the grant of the patent: **14.10.2015**
(21) Application number: 09707226.8
(22) Date of filing: 05.02.2009
(51) Int. Cl.: A61K 38/13, A61P 21/00

(54) **NON-IMMUNOSUPPRESSIVE CYCLOSPORIN FOR THE TREATMENT OF LIMB-GIRDLE MUSCULAR DYSTROPHY**
NICHT IMMUNSUPPRESSIVES CYCLOSPORIN ZUR BEHANDLUNG VON GLIEDERGÜRTEL-MUSKELDYSTROPHIE
CYCLOSPORINE NON IMMUNOSUPPRESSIVE POUR LE TRAITEMENT DE LA DYSTROPHIE MUSCULAIRE DES CEINTURES

(30) Priority: 08.02.2008 WO PCT/IB2008/000292
(43) Date of publication of application: 17.11.2010
(73) Proprietor: DEBIOPHARM S.A., 1002 Lausanne (CH)
(72) Inventor: MOLKENTIN, Jeffery, D., Cincinnati OH 45247 (US)
(74) Representative: Grosfillier, Philippe
(86) International application number: PCT/IB2009/000204
(87) International publication number: WO 2009/098577

(56) References cited:
- WO-A-2006/072639
- ANGELIN A ET AL: "Mitochondrial dysfunction in the pathogenesis of Ullrich congenital muscular dystrophy and prospective therapy with cyclosporins" PROCEEDINGS OF THE NATIONAL ACADEMY OF SCIENCES OF THE UNITED STATES OF AMERICA 20070116 US, vol. 104, no. 3, 16 January 2007 (2007-01-16), pages 991-996, XP002504969 ISSN: 0027-8424
- EMERY A E: "The muscular dystrophies" LANCET THE, LANCET LIMITED. LONDON, GB, vol. 359, no. 9307, 23 February 2002 (2002-02-23), pages 687-695, XP004790771 ISSN: 0140-6736
- MAGNUS J HANSSON ET AL: "The Nonimmunosuppressive Cyclosporin Analogs NIM811 and UNIL025 Display Nanomolar Potencies on Permeability Transition in Brain-Derived Mitochondria" JOURNAL OF BIOENERGETICS AND BIOMEMBRANES, KLUWER ACADEMIC PUBLISHERS-PLENUM PUBLISHERS, NE, vol. 36, no. 4, 1 August 2004 (2004-08-01) , pages 407-413, XP019280564 ISSN: 1573-6881
- PARSONS STEPHANIE A ET AL: "Genetic disruption of calcineurin improves skeletal muscle pathology and cardiac disease in a mouse model of limb-girdle muscular dystrophy." THE JOURNAL OF BIOLOGICAL CHEMISTRY 30 MAR 2007, vol. 282, no. 13, 30 March 2007 (2007-03-30), pages 10068-10078, XP002504970 ISSN: 0021-9258 cited in the application
- DANIELE ET AL: "Ins and outs of therapy in limb girdle muscular dystrophies" INTERNATIONAL JOURNAL OF BIOCHEMISTRY AND CELL BIOLOGY, EXETER, GB, vol. 39, no. 9, 1 January 2007 (2007-01-01), pages 1608-1624, XP022168756 ISSN: 1357-2725
- NAKAYAMA HIROYUKI ET AL: "Ca2+- and mitochondrial-dependent cardiomyocyte necrosis as a primary mediator of heart failure." THE JOURNAL OF CLINICAL INVESTIGATION SEP 2007, vol. 117, no. 9, September 2007 (2007-09), pages 2431-2444, XP002572910 ISSN: 0021-9738
- MILLAY DOUGLAS P ET AL: "Genetic and pharmacologic inhibition of mitochondrial-dependent necrosis attenuates muscular dystrophy." NATURE MEDICINE APR 2008, vol. 14, no. 4, April 2008 (2008-04), pages 442-447, XP002572911 ISSN: 1546-170X
- REUTENAUER J ET AL: "Investigation of Debio 025, a cyclophilin inhibitor, in the dystrophic mdx mouse, a model for Duchenne muscular dystrophy." BRITISH JOURNAL OF PHARMACOLOGY OCT 2008, vol. 155, no. 4, October 2008 (2008-10), pages 574-584, XP002572912 ISSN: 0007-1188

## Description

The present invention relates to the use of a non-immunosuppressive cyclosporin A derivative for reducing the induction of myofiber necrosis and myofiber degeneration in a subject diagnosed with Limb-Girdle Muscular Dystrophy (LGMD), in particular sarcoglycanopathy.

Muscular dystrophies (MD) comprise a diverse group of inherited disorders that largely affect striated muscle tissue resulting in progressive muscle weakness, wasting, and in many instances, premature death. Many characterized mutations that are causally linked to MD in humans are associated with alterations in structural attachment proteins that affix the underlying contractile proteins to the basal lamina, providing rigidity to the skeletal muscle cell membrane (sarcolemma), or in proteins that directly stabilize or repair the cell membrane, such as e.g. sarcoglycan or dystrophin.

Inherited mutations of sarcoglycan genes alpha-, beta-, gamma- and delta-sarcoglycan genes) cause a skeletal muscle disease with heterogeneous syndromes, LGMD and a subgroup thereof, the sarcoglycanopathies. The syndromes or phenotypes of the sarcoglycanopathies depend on which sarcoglycan gene is mutated and the type of genetic mutations (allelic variant), and show four forms of specific disorders: LGMD types 2C, 2D, 2E and 2F (mutations of gamma-, alpha-, beta- and delta-sarcoglycan genes, respectively) which represent 25% of all diagnosed LGMD cases. In particular, the different mutations specifically observed in the delta-sarcoclycan gene result in the severe disorder of LGMD type 2F or LGMD2F (Online Mendelian Inheritance in Man [OMIM] #601287, genetic mutations: [OMIM] 601411. Emery et al., The Lancet, 2002, 359:687-695.).

The different LGMD types are characterized by progressive wasting and weakness with atrophy predominantly involving muscles of arms and legs proximal to shoulder and hip, respectively. Disease phenotypes resemble those of severe Duchenne-like or Becker-like muscular dystrophy syndromes. However, the latter diseases involve different molecular mechanisms and genetic disorders. Before molecular diagnoses of LGMD were available, LGMD patients were often diagnosed as suffering from Duchenne muscular dystrophy. The onset of the disease varies from early childhood to adulthood with mild to severe clinical forms. Up to 25% of patients show severe forms of the disease, developing severe lumbar lordosis, contractures of the Achilles tendons, muscle hypertrophy, cardiomyopathy and cardiac conduction defects. Hypertrophy of the calves or tongue, selectivity of muscle involvement and late stage cardiac complications are associated more or less specifically with each of the different forms (Danièle et al., Int J. Biochem Cell Biol., 2007;39:1608-1624). Progressive weakness leads to restrictive lung disease and hypoventilation requiring ventilatory assistance. In general, morbidity and mortality rates vary. With early onset, progression of the disease to death typically is very rapid. Death is often a consequence of respiratory complications.

To date, no specific treatment is available for patients suffering from any of the LGMD syndromes. Aggressive supportive care, e.g., orthopedy, surgery and physical therapy for preserving muscle function, maximizes functional ability and prolongs life expectancy. These measures, however, cannot prevent myofiber degeneration and the eventual occurrence of respiratory complications.

With the development of animal models lacking specific genes involved in muscular dystrophy, a better understanding of the molecular mechanisms underlying the sarcoglycanopathies has been forthcoming. Recently, it was shown that muscle cells of mice lacking delta-sarcoglycan (*scgd*-/- mice) due to targeted inactivation of the delta-sarcoglycan gene (*scgd*) have a propensity for increased cellular calcium influx. It is assumed that loss of components of the dystrophin-glycoprotein complex (DGC) such as dystrophin or sarcoglycan complex results in a fundamental alteration of the physical properties of the muscle cell membrane and in increased permeability and leakiness of the sarcolemma. Activation of unregulated calcium influx channels caused by membrane instability and fragility may initiate dystrophic disease in skeletal muscle leading to myofiber degeneration and induction of myofiber necrosis.

Parsons et al. (J. Biol. Chem., 2007, 282:10068-10078) showed that inhibition of calcium/calmodulin-activated serine/threonine protein phosphatase calcineurin (calcineurin) activity by genetic deletion decreased skeletal muscle and myofiber degeneration and inflammation in *scgd-*/*-* mice, and was cytoprotective. Analogous disease improvements following inhibition of calcineurin activity were not observed in mice lacking the *mdx* gene (dystrophin gene mutation) used as model for Duchenne muscular dystrophy even though increased calcium influx was observed in both muscular dystrophy models. In fact, an activated transgene for calcineurin protected mdx mice (Chakkalakal et al., Hum. Mol. Genet., 2004, 13: 379-399; Stupka et al. Acta Neuropathol., 2004, 107: 299-310, but see De Luca et al., Am. J. Pathol., 2005, 166: 477-489). Parsons et al. identified calcineurin as a potential target for LGMD therapeutics. Based on their observations, Parsons et al. suggested that inhibition of calcineurin activity may provide some benefit in select types of muscle disease, such as limb-girdle muscular dystrophy and that, therefore, cyclosporin A (CsA) could be potentially beneficial.

The effector mechanisms that cause progressive myofiber degeneration induced by altered membrane permeability in LGMD are not well understood and are subjects of active investigations which may provide a basis for the eventual development of mechanism-based novel treatment strategies for patients suffering from this disorder. At this time, however, there is no effective method available for the treatment of patients suffering from LGMD. Hence there is a need for new therapeutic approaches such as that described herein.

The aim of the present invention is to provide the clinician with a therapy for the treatment of the induction and the progression of myofiber necrosis and muscle degeneration in a patient suffering from LGMD and, in particular, from a sarcoglycanopathy, and more particularly from LGMD type 2F. This therapy should protect against necrosis of dystrophic skeletal muscles and also on cardiac and diaphragm muscle and should delay disease progression.

The present inventors surprisingly found that the administration of a non-immunosuppressive cyclosporin A (CsA) derivative, which does not inhibit calcineurin, to a subject suffering from disorders diagnosed as LGMD, and, in particular, from sarcoglycanopathy, and more particularly from LGMD type 2F, is an effective therapy. They observed that administration of non-immunosuppressive CsA derivative [D-MeAla]³-[EtVal]⁴-CsA reduces muscle pathology of a subject diagnosed with myofiber necrosis, in particular with LGMD, and more particularly with LGMD type 2F, and degeneration and progression of the disease, as well as normalizes myofiber area distribution by reducing the sensitivity of mitochondria to the latent calcium overload.

A subject may be a human being or a mammal such as, e.g., a mouse that shows a muscular dystrophy phenotype owing to the deletion of a gene or non-expression of a gene responsible for the phenotype of the disease.

Therefore, the present invention relates to The non-immunosuppressive CsA derivative suitable for use with the present invention was also described in International Patent Application WO 2005/021028 by Novartis AG, on pages 3-6. [D-MeAla]³-[EtVal]⁴-CsA was disclosed by Wenger et al. in International Patent Application WO 00/01715. [D-MeAla]³-[EtVal]⁴-CsA of formula III has been attributed the CAS Registry Number 254435-95-5.

The non-immunosuppressive CsA derivative for use in the present invention is cyclic undecapeptide described by the following formula: 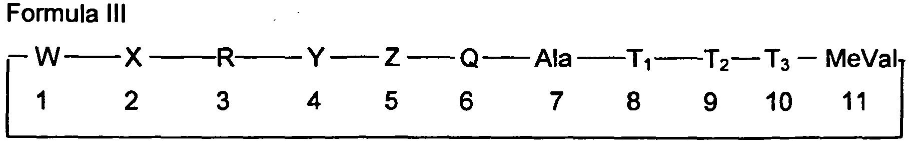 wherein
W is MeBmt;
X is αAbu;
R is (D)-MeAla;
Y is N-ethylVal (EtVal);
Z is Val;
Q is MeLeu;
T₁ is (D)Ala;
T₂ is MeLeu ; and
T₃ is MeLeu,
and where MeBmt is N-methyl-(4R)-4-but-2E-en-1-yl-4-methyl-(L)threonine, αAbu is L-α-aminobutyric acid, D-MeAla is N-methyl-D-alinine, EtVal is N-ethyl-L-valine, Val is L-valine, MeLeu is N-methyl-L-leucine, Ala is L-alanine, (D)Ala is D-alanine, and MeVal is N-methyl-L-valine. The conventional numbering of amino acid positions generally used in reference of Cyclosporin A is shown below the formula. Composite names are used for derivatives of CsA, the composite names comprising a first portion indicating the identity of residues that are different from those in cyclosporin A and providing their position, and a second portion labelled "CsA" indicating that all other residues are identical to those in Cyclosporin A. For example, [Melle]⁴-CsA is a cyclosporin that is identical to cyclosporin A except that MeLeu in position 4 is replaced by Melle (N-methyl-L-isoleucine).

In a further embodiment, the invention relates to a non-immunosuppressive CsA derivative [D-MeAla]³-[EtVal]⁴-CsA of formula III, for use in treatment of LGMD, and, in particular, from sarcoglycanopathy, and more particularly from LGMD type 2F.

In another embodiment, the invention relates to a cyclic undecapeptide according to claim 1 for use in a method for preventing or reducing muscular degeneration in a subject suffering from LGMD, and, in particular, from sarcoglycanopathy, and more particularly from LGMD type 2F,comprising administering to the subject an effective amount of a non-immunosuppressive CsA derivative [D-MeAla]³-[EtVal]⁴-CsA of formula III. An effective amount of a non-immunosuppressive cyclosporin A derivative is understood to be an amount that when administered repeatedly in the course of a therapeutic regimen to a subject suffering from LGMD, and, in particular, from sarcoglycanopathy, and more particularly from LGMD type 2F results in an objective clinical response such as an improvement, stabilization or slow-down in the progression of the disease. When administered orally, an effective amount for daily or trice weekly administration will be between about 1 mg/kg (body weight) to about 100 mg/kg, preferably from about 1 mg/kg to about 20 mg/kg. By intravenous route, the indicated corresponding dosage may be from about 1 mg/kg to about 50 mg/kg, preferably from about 1 mg/kg to about 25 mg/kg.

Moreover, the invention relates to a pharmaceutical composition for use in preventing or reducing muscular degeneration in a subject suffering from LGMD, and, in particular, from sarcoglycanopathy, and more particularly from LGMD type 2Fcomprising an effective amount of a non-immunosuppressive CsA derivative [D-MeAla]³-[EtVal]⁴-CsA of formula III, a pharmaceutically acceptable carrier and, optionally, an excipient and a diluent. The diluent typically is water. Excipients that are typically added to parenteral formulations include an isotonic agent, a buffer or other pH-controlling agent, and a preservative. The compositions may comprise other active ingredients such as an antibiotic, a glucocorticoid , a corticosteroid such as, e.g., prednisone.

The present invention will be explained further below with the aid of the following drawings.

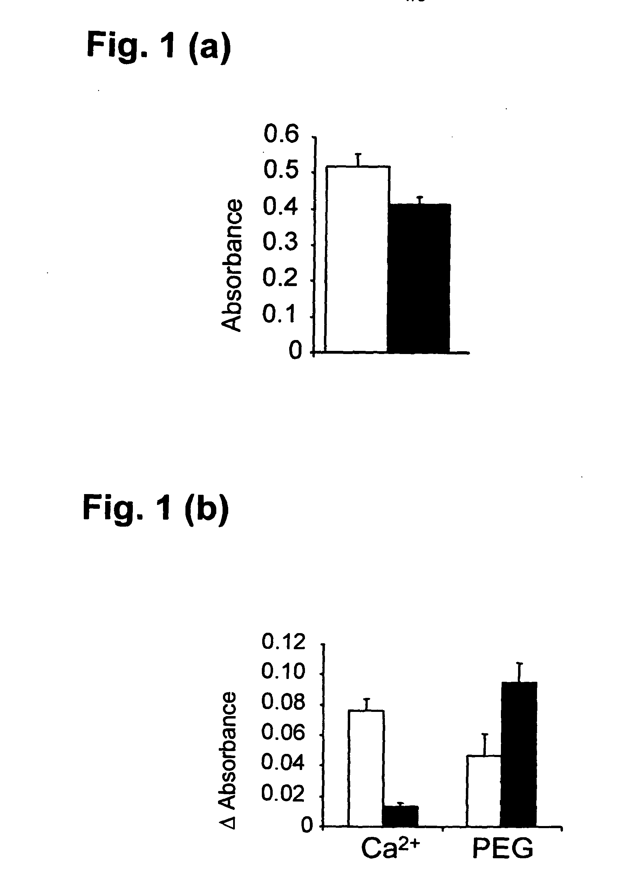

- Fig. 1 (a) represents baseline swelling, measured as absorbance at 540 nm, of mitochondria from skeletal muscle of 6 week-old wildtype mice (Wt) (white bar) and *scgd-*/*-* mice (black bar). Mitochondria from the plantar muscle group, quadriceps, and tibialis anterior were combined. As indicated by the lower absorbance measurement, mitochondria from *scgd-*/*-* mice muscle were more swollen at baseline than mitochondria from Wt mice.
- Fig 1 (b) represents changes in mitochondrial swelling after 10 minutes of treatment with calcium (Ca2+) or PEG-3350 (PEG) measured as differences of absorbance at 540 nm between untreated and treated mitochondria from 6 week-old wildtype mice (Wt) (white bar) and s*cgd-*/*-* mice (black bar). Mitochondria from the plantar muscle group, quadriceps, and tibialis anterior were combined.

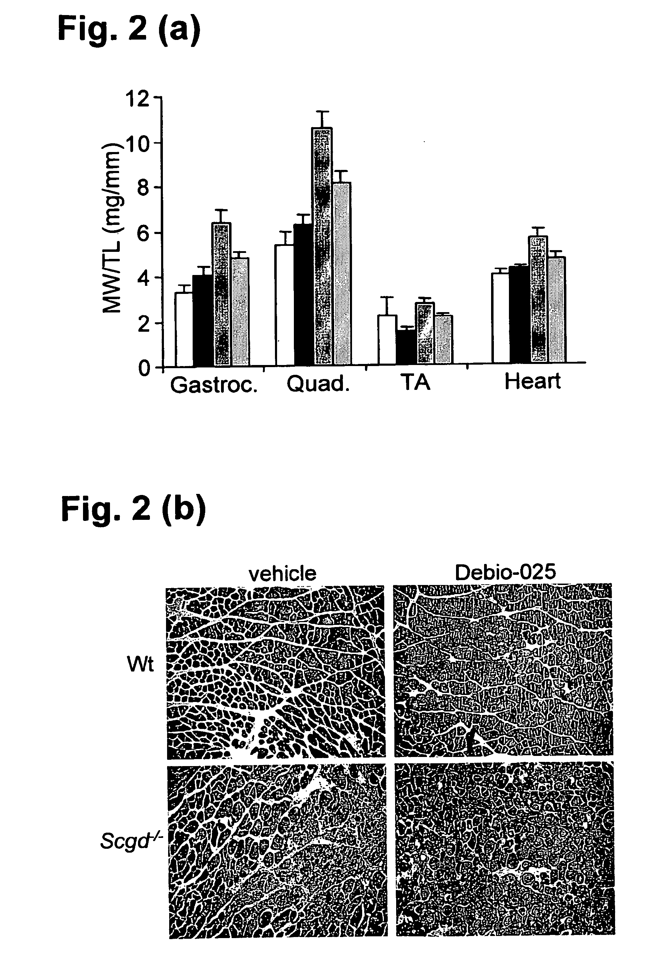

- Fig. 2(a) represents reduction of muscle pathology after the administration of D-[MeAla]³-[EtVal]⁴-CsA in *scgd-*/*-* mice. Muscle weight (MW)-to-tibia length (TL) ratios (MW/TL) were measured in gastrocnemius (Gastroc.), quadriceps (Quad.), tibialis anterior (TA), and heart muscle from wild type (Wt) mice treated with either vehicle (white bar) or D-[MeAla]³-[EtVal]⁴-CsA (black bar) or from *scgd-*/*-* mice treated with either vehicle (gray bar or second to last bar in group) or D-[MeAla]³-[EtVal]⁴-CsA (dotted bar or last bar of group). D-[MeAla]³-[EtVal]⁴-CsA prevents an increase in muscle weight in Scgd-/- mice, which increase is associated with disease.
- Fig. 2(b) represents reduction of muscle pathology after administration of D-[MeAla]³-[EtVal]⁴-CsA in Scgd-/- mice observed in representative hematoxylin- and eosin-stained sections of quadriceps from wild type (Wt) mice and *scgd-l-*mice treated with D-[MeAla]³-[EtVal]⁴-CsA. Corresponding vehicle controls are also shown.

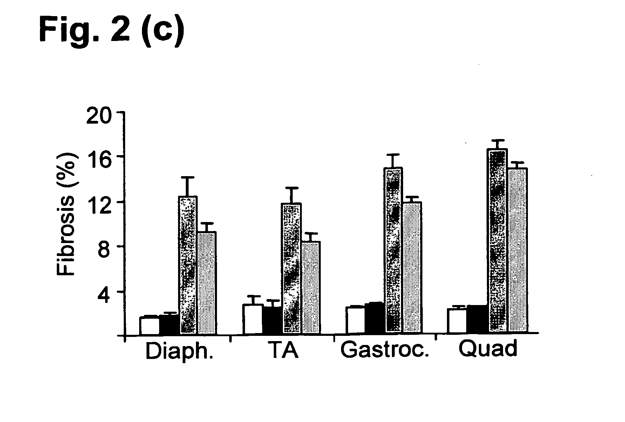

- Fig. 2(c) represents reduction of muscle pathology after administration of D-[MeAla]³-[EtVal]⁴-CsA in *scgd-*/*-* mice as assessed by quantitation of fibrotic areas in trichrome-stained sections from diaphragm (Diaph.), tibialis anterior (TA), gastrocnemius (Gastroc.), quadriceps (Quad). Assessed were sections from wild type (Wt) mice treated with either vehicle (white bar) or D-[MeAla]³-[EtVal]⁴-CsA (black bar) and from *scgd-*/*-* mice treated with either vehicle (gray bar or second to last bar in group) or D-[MeAla]³-[EtVal]⁴-CsA (dotted bar or last bar of group). D-[MeAla]³-[EtVal]⁴-CsA reduced fibrosis in *scgd*-/- mice.

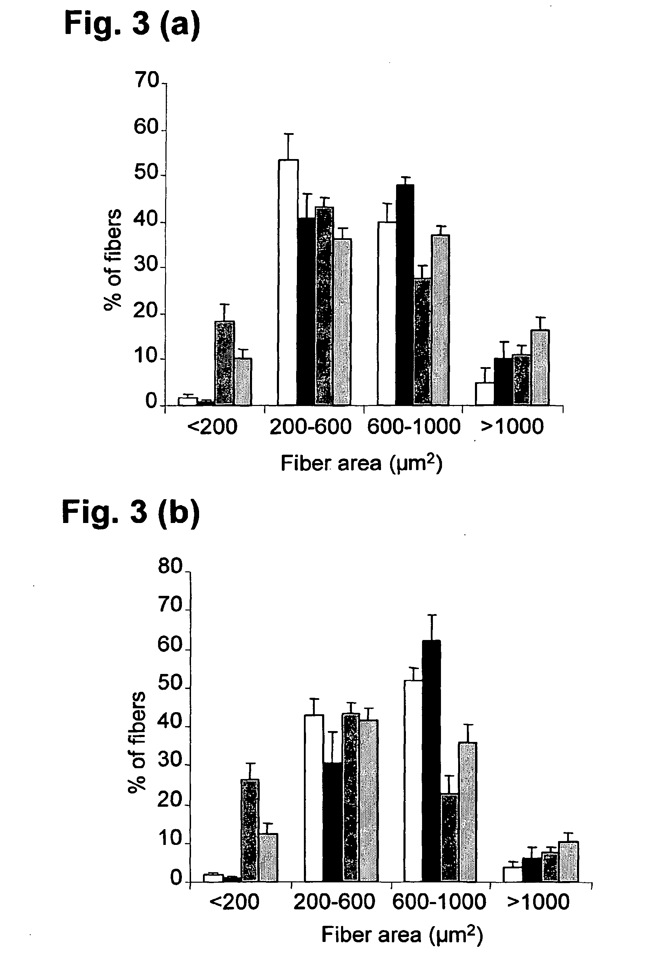

- Fig. 3(a) represents reduction of fiber area heterogeneity in muscle from *scgd-*/*-* mice after the administration of D-[MeAla]³-[EtVal]⁴-CsA as assessed by quantitation of the distribution of fiber areas in tibialis anterior (muscle). The study included wild type (Wt) mice treated with either vehicle (white bar) or D-[MeAla]³-[EtVal]⁴-CsA (black bar) and *scgd-*/*-* mice treated with either vehicle (gray bar or second to last bar in group) or D-[MeAla]³-[EtVal]⁴-CsA (dotted bar or last bar of group). ("<" means "inferior to", ">" means "superior to"). D-[MeAla]³-[EtVal]⁴-CsA treatment normalized fiber area heterogeneity in *scgd-*/*-* mice.
- Fig. 3(b) represents reduction of fiber area heterogeneity in muscle from *scgd-*/*-* mice after the administration of D-[MeAla]³-[EtVal]⁴-CsA as assessed by quantitation of the distribution of fiber areas in gastrocnemius. Included in the study were wild type (Wt) mice treated with either vehicle (white bar) or D-[MeAla]³-[EtVal]⁴-CsA (black bar) and *scgd-*/*-* mice treated with either vehicle (gray bar or second to last bar in group) or D-[MeAla]³-[EtVal]⁴-CsA (dotted bar or last bar of group). ("<" means "inferior to", ">" means "superior to"). D-[MeAla]³-[EtVal]⁴-CsA treatment normalized fiber area heterogeneity in *scgd-*/*-* mice.

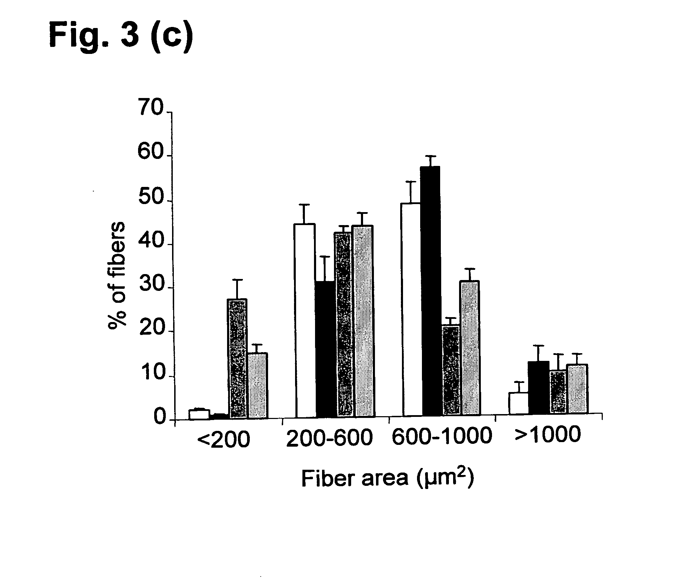

- Fig. 3(c) represents reduction of fiber area heterogeneity in muscle from *scgd-*/*-* mice after the administration of D-[MeAla]³-[EtVal]⁴-CsA as assessed by quantitation of the distribution of fiber areas in quadriceps muscle. Measurements were carried out on wild type (Wt) mice treated with either vehicle (white bar) or D-[MeAla]³-[EtVal]⁴-CsA (black bar) and *scgd-*/*-* mice treated with either vehicle (gray bar or second to last bar in group) or D-[MeAla]³-[EtVal]⁴-CsA (dotted bar or last bar of group). ("<" means "inferior to", ">" means "superior to"). D-[MeAla]³-[EtVal]⁴-CsA treatment normalized fiber area heterogeneity in *scgd-*/*-* mice.

If enhanced calcium concentration serves as an initiator of LGMD through myofiber necrosis, a number of downstream calcium-dependent effectors can be potentially implicated as causative factors. For example, increased calcium can lead to myotube necrosis through activation of the calcium-activated protease calpain, a signalling protein critically involved in skeletal muscle regeneration after injury and the differentiation of skeletal muscle cells.

Parsons et al. (2007) showed in a mouse model of LGMD that inhibition of calcium/calmodulin-activated serine/threonine protein phosphatase calcineurin (calcineurin) activity by genetic deletion decreased skeletal muscle and myofiber degeneration and inflammation, i.e., improved skeletal muscle pathology. This was not the case in a mouse model of Duchenne wherein the inhibition of calcineurin by CsA was deleterious for muscle pathology (Stupka et al., Acta Neuropathol, 2004, 107:299-310). This discrepancy in the consequences of inhibition of calcineurin activity in different dystrophies characterized by elevated calcium concentrations in muscle cells is presumably due to their respective different genetic deletions involving and affecting different alterations of the muscle cell membrane and different signalling pathways.

Another major mechanism leading to cellular necrosis is mitochondrial calcium overload, which secondarily enhances reactive oxygen species (ROS) generation and further promotes MPT (Mitochondrial Permeability Transition). Increased subsarcolemmal calcium can also promote a local increase in reactive oxygen species (ROS) leading to greater defects in the cell membrane and additional calcium entry, further promoting cellular necrosis and/or apoptosis.

Experiments were carried out to experimentally test the existence of a causal link between the lack of the *scgd* gene, the progressive degeneration of myofibers, and cellular necrosis and/or apoptosis induced by mitochondrial dysfunction.

To assess whether calcium-induced mitochondrial dysfunction can initiate and drive the progressive degeneration of myofibers associated with LGMD, the present inventors compared mitochondria isolated from dystrophic skeletal muscle of *scgd* -/- mice and wild type mice. Mitochondria isolated by homogenization in a sucrose-containing buffer (250 mM sucrose, 10 mM Tris (pH 7.4), 1 mM EDTA) from the plantar muscle group, quadriceps, and tibialis anterior were suspended after washes and centrifugation in an isotonic buffer (120 mM KCI, 10 mM Tris (pH 7.4), 5mM KH₂PO₄) and tested in a swelling assay. This assay consisted of incubation of isolated mitochondria with 200 µM CaCl₂ (swelling) or 5%(w/v) PEG-3350 (shrinkage). Swelling produces a reduction and shrinkage an increase in absorbance at 540 nm. Results are presented as means ± SEM (standard error of the means) (Fig. 1 (a) and Fig. 1 (b)). A two-sample Student t test was used, and values were only considered significant when p<0.05.

Mitochondria isolated from skeletal muscle of *scgd-*/*-* mice were swollen at baseline compared to those of wild type mice. They were also refractory to additional swelling by exogenously applied calcium and did not show a reversal in baseline swelling (Fig. 1(a) and Fig. 1(b)). This insensitivity of mitochondria of *scgd -*/*-* mice to additional calcium indicated that they were swollen and pathological, consistent with downstream pathological effects such as a pseudo-hypertrophy response in gastrocnemius and quadriceps at 6 weeks of age, which hypertrophy is associated with tissue inflammation, a profound reduction in muscle weights with aging compared to the wild type mice, a characteristic increase in central nucleation of myofibers indicating regeneration due to ongoing degeneration and numerous degeneration/regeneration cycles, and a destabilization of muscle membrane damaged by calcium excess.

The above findings strongly suggest that a calcium-dependant MPT process is underlying the progressive degeneration of myofibers associated with LGMD, even though the latent mitochondrial abnormality may not be predictive of the severity of the clinical syndrome. In principle, the pathogenic chain of events downstream of the genetic lesion may be interrupted by an appropriate drug. For example, loss of myostatin activity can ameliorate LGMD in *scgd-*/*-* mice by reducing fibrosis and inducing muscle regeneration. Inhibition of calcineurin activity was reported to produce analogous effects. According to the above-discussed new findings and those presented under Examples 1 and 2, modulation of degeneration/regeneration cycles and reduction of degeneration of skeletal muscle in a mouse model of LGMD can be achieved through normalization of mitochondrial function. These findings of the inventors enable a new pharmacological treatment of patients affected by LGMD, which treatment targets mitochondrial function but not calcineurin activity and does not cause immunosuppression.

Accordingly, the present invention relates to the use of the non-immunosuppressive CsA derivative D-[MeAla]³-[EtVal]⁴-CsA, for preventing or reducing muscular degeneration in a subject suffering from LGMD. The non-immunosuppressive CsA derivative can also be used for normalizing mitochondrial function in mitochondria prepared from muscle biopsies of a subject suffering from LGMD. A finding of tolerance to calcium overload in such mitochondria will serve as an indicator that treatment of the patient with a non-immunosuppressive CsA derivative will be effective in reducing the severity of the disease.

The active compound, i.e., the non-immunosuppressive cyclosporin A derivative used for treating patients suffering from LGMD, may be administered by any conventional route. It may be administered parentally, e.g., in the form of injectable solutions or suspensions, or in the form of injectable deposit formulations. Preferably, it will be administered orally in the form of solutions or suspensions for drinking, tablets or capsules. Pharmaceutical compositions for oral administration comprising non-immunosuppressive cyclosporin A derivative [D-MeAla]3-[EtVal]4-CsA are described in Examples. Such pharmaceutical compositions typically comprise the non-immunosuppressive cyclosporin A derivative of choice and one or more pharmaceutically acceptable carrier substances. Suitable pharmaceutical carriers are described, e.g., in Remington's Pharmaceutical Sciences, 17th ed., Mack Publishing Company, Easton, PA (1990), which is a standard reference text in this field. Typically, these compositions are concentrated and need to be combined with an appropriate diluent, e.g., water, prior to administration. Pharmaceutical compositions for parenteral administration typically also include one or more excipients. Optional excipients include an isotonic agent, a buffer or other pH-controlling agent, and a preservative. These excipients may be added for maintenance of the composition and for the attainment of preferred ranges of pH (about 6.5-7.5) and osmolarity (about 300 mosm/L).

Additional examples of cyclosporin formulations for oral administration can be found in U.S. Pat. Nos. 5,525,590 and 5,639,724, and U.S. Pat. Appl. 2003/0104992. By the oral route, the indicated dosage of a non-immunosuppressive cyclosporin A derivative for daily to trice weekly administration may be from about 1 mg/kg (body weight) to about 100 mg/kg, preferably from about 1 mg/kg to about 20 mg/kg. By the intravenous route, the indicated corresponding dosage may be from about 1 mg/kg to about 50 mg/kg, preferably from about 1 mg/kg to about 25 mg/kg. An effective amount of the non-immunosuppressive cyclosporin A derivative is understood to be an amount that when administered repeatedly in the course of a therapeutic regimen to a LGMD patient results in an objective clinical response such as an improvement, stabilization or slow-down in the progression of the disease. Such clinical response can be assessed, e.g., by Quantitative Isometric Strength (QIS) testing. QIS allows evaluation of muscle strength in an objective way with the aid of pressure-transducing and recording equipment. Alternatively, normalization of rates of apoptosis can be assessed in muscle biopsies by biochemical and immunohistochemical methods known to the person skilled in the art. Finally, electromyography may be utilized that shows a muscular instead of a neurogenic pattern, which may be quantified.

Numerous factors will be taken into consideration by a clinician when determining trial doses for testing efficacy of a pharmaceutical composition of the invention comprising the non-immunosuppressive cyclosporin A derivative [D-MeAla]3-[EtVal]4-CsA. Primary among these are the toxicity and half-life of the non-immunosuppressive cyclosporin A derivative. Additional factors include the size of the patient, the age of the patient, the general condition of the patient (including mechanical ventilation, clinical stage of the disease, the severity of the symptoms), the presence of other drugs in the patient, and the like. A course of treatment will require repeated administration of a pharmaceutical composition of the invention. Typically, an adequate drug dose will be administered about once per day. Because of the genetic nature of the disease, treatment may need to be continued for an extended period of time, possibly for the life of the patient.

No effective pharmacological treatment of LGMD is presently known. Patients are supported by vaccination against influenza and pneumococal infection, and any infection is treated aggressively with antibiotics. Hence, pharmaceutical compositions of the present invention may comprise one or more other active ingredients in addition to the non-immunosuppressive cyclosporin A derivative such as, for example, one or more antibiotics. The non-immunosuppressive cyclosporin A derivative and such other active ingredient can be administered together as part of the same pharmaceutical composition or can be administered separately as part of an appropriate dose regimen designed to obtain the benefits of all active ingredients. The appropriate dose regimen, the amount of each dose administered, and specific intervals between doses of each active agent will depend upon the specific combination of active agents employed, the condition of the patient being treated, and other factors discussed in the previous section. Such additional active ingredients will generally be administered in amounts equal to those for which they are known to be effective as single therapeutic agents. The FDA approved dosages for such active agents that have received FDA approval for administration to humans are publicly available.

The invention is further elaborated by the following examples. The examples are provided for purposes of illustration to a person skilled in the art, and are not intended to be limiting the scope of the invention as described in the claims. Thus, the invention should not be construed as being limited to the examples provided.

### Examples:

### Example 1: Stabilization of calcium-induced damage in muscle membrane and reduction of muscular dystrophy progression and pathology

To stabilize calcium-induced damage to muscle membrane and reduce disease progression caused by numerous degeneration/regeneration cycles, *scgd-*/*-* mice were treated with D-[MeAla]³-[EtVal]⁴-CsA.

*Scgd-*/*-* mice were treated by subcutaneous administration of either D-[MeAla]³-[EtVal]⁴-CsA at a dose of 50 mg/kg/day or vehicle (formulation which does not contain the active ingredient D-[MeAla]³-[EtVal]⁴-CsA), beginning at 4 weeks of age and ending at 10 weeks of age. Different dissected muscles, i.e., gastrocnemius, quadriceps, tibialis anterior and heart muscles, were weighed and muscle weight (MW)-to-tibia length (TL) ratios (MW/TL) were determined. Results in Fig. 2 (a) and Fig. 2 (c) are presented as means ± SEM (standard error of the means). A one-way ANOVA was used to compare means among 3 or more independent groups. A Newman-Keuls post hoc test was applied whenever multiple comparisons were conducted using InStat 3.0 (GraphPad software from Science Inc.). Values were considered significant when p<0.05.

In response to the numerous degeneration/regeneration cycles, *scgd-*/*-* mice initially presented with hypertrophic skeletal muscles as are seen in subjects suffering from LGMD. Administration of D-[MeAla]³-[EtVal]⁴-CsA in *scgd-*/*-* mice resulted in a reduction in muscle pathology observed as a reduction of pseudo-hypertrophy responses in skeletal muscles of treated *scgd-*/*-* mice (Fig. 2 (a)). Reduction of skeletal muscular hypertrophy was about 1.3 fold in gastrocnemius (Gastroc.), quadriceps (Quad.)and tibialis anterior (TA) and about 1.2 fold in heart muscle (Heart) of D-[MeAla]³-[EtVal]⁴-CsA-treated *scgd-*/*-* mice compared to vehicle-treated animals.

Reduced pathology in the *scgd-*/*-* mice treated with D-[MeAla]³-[EtVal]⁴-CsA was also observed in histological slides of quadriceps (Fig. 2 (b)). Improvement in myofiber organization and normalization of fiber area distributions were noticed in histological slides from D-[MeAla]³-[EtVal]⁴-CsA-treated *scgd-*/*-* mice over vehicle-treated wild type mice or *scgd-*/*-* mice.

Percentage fibrosis was assessed by means of a biochemical assay that quantified hydroxyproline content (Parsons et al., Am. J. Pathol., 2006, 168:1975-1985) in diaphragm (Diaph.), tibialis anterior (TA), gastrocnemius (Gastroc.) and quadriceps (Quad) muscles from wild type and *scgd-*/*-* mice treated or not treated with D-[MeAla]³-[EtVal]⁴-CsA. Treatment of the Scgd-/- mice with D-[MeAla]³-[EtVal]⁴-CsA reduced fibrosis in the different muscles - by about 1,5% to 3,5% (Fig 2 (c)).

### Example 2: Normalization of distribution of small and large fibers and reduction of degeneration/regeneration cycles in skeletal muscles of scgd-/- mice after treatment with D-[MeAla]³-[EtVal]⁴-CsA

Treatment with D-[MeAla]³-[EtVal]⁴-CsA of *scgd-*/*-* mice partially normalized fiber area distributions in tibialis anterior (Fig. 3(a)), gastrocnemius (Fig. 3(b)) and quadriceps muscles (Fig. 3(c)), indicating a reduction of degeneration/regeneration cycles. *Scgd-*/*-* mice were administered subcutaneously with either D-[MeAla]³-[EtVal]⁴-CsA at a dose of 50 mg/kg/day or vehicle for 6 weeks. Histological analysis of the fibers of the three skeletal muscles from *scgd-*/*-* mice showed significant increases in populations of small-diameters fiber (<200 µm²) relative to the wild type mice, indicating an increased number of regenerating fibers, ongoing degeneration/regeneration cycles and disease progression. The administration of the non-immunosuppressive D-[MeAla]³-[EtVal]⁴-CsA to *scgd-*/*-* mice reduced the regeneration rate of the fibers and normalized partially the distribution of small and large fibers. Less small fibers were observed in *scgd-*/*-* mice treated by non-immunosuppressive D-[MeAla]³-[EtVal]⁴-CsA compared to the *scgd-*/*-* mice that only received vehicle.

### Example 3: Oral formulations of [D-MeAla]³-[EtVal]⁴-CsA.

Amounts are expressed as % w/w.

### Example A:

| | |
|---|---|
| [D-MeAla]³-[EtVal]⁴-CsA | 10 |
| Glycofurol 75 | 35.95 |
| Miglycol 812 | 18 |
| Cremophor RH40 | 35.95 |
| Alpha-Tocopherol | 0.1 |

### Example B:

| | |
|---|---|
| [D-MeAla]³-[EtVal]⁴-CsA | 10 |
| Tetraglycol | 2 |
| Captex 800 | 2 |
| Nikkol HCO-40 | 85.9 |
| Butylhydroxytoluene (BHT) | 0.1 |

### Example C:

| | |
|---|---|
| [D-MeAla]³-[EtVal]⁴-CsA | 10 |
| Glycofurol 75 | 39.95 |
| Miglycol 812 | 14 |
| Cremophor RH40 | 36 |
| Butylhydroxyanisole (BHA) | 0.05-0.1 |

### Example D:

| | |
|---|---|
| [D-MeAla]³-[EtVal]⁴-CsA | 10 |
| Tetraglycol | 10 |
| Myritol | 5 |
| Cremophor RH40 | 74.9 |
| Alpha-Tocopherol | 0.1 |

### Example E:

| | |
|---|---|
| [D-MeAla]³-[EtVal]⁴-CsA | 10 |
| Ethanol | 9 |
| Propylene glycol | 8 |
| Cremophor RH40 | 41 |
| Glycerol monolinoleate | 32 |

For individual components of formulations A-D and for methods of preparation see British Patent Appl. No. 2,222,770.

## Claims

1. A cyclic undecapeptide of the following formula: 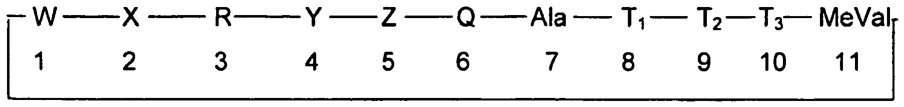 wherein
W is MeBmt;
X is αAbu;
R is (D)-MeAla; Y is N-ethylVal (EtVal); Z is Val; Q is MeLeu;
T₁ is (D)Ala;
T₂ is MeLeu; and
T₃ is MeLeu
for use in treatment of Limb-Girdle Muscular Dystrophy.

## Patentansprüche

1. Cyclisches Undecapeptid der folgenden Formel: 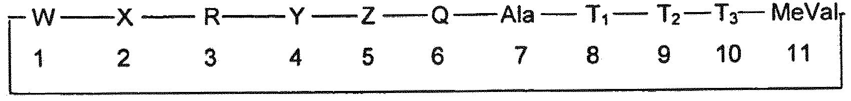 wobei
W für MeBmt steht;
X für αAbu steht;
R für (D) -MeAla steht;
Y für N-EthylVal (EtVal) ;
Z für Val steht;
Q für MeLeu steht;
T₁ für (D) Ala steht;
T₂ für MeLeu steht und
T₃ für MeLeu steht;
zur Verwendung bei der Behandlung von Gliedergürtel-Muskeldystrophie.

## Revendications

1. Undécapeptide cyclique de formule suivante : 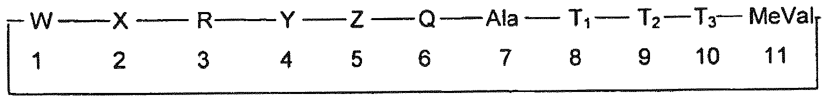 dans laquelle
W est MeBmt ;
X est αAbu ;
R est (D)-MeAla ;
Y est N-éthylVal (EtVAl) ;
Z est Val ;
Q est MeLeu ;
T₁ est (D)Ala ;
T₂ est MeLeu ; et
T₃ est MeLeu
pour une utilisation dans le traitement de la Dystrophie Musculaire Scapulo-Humérale.
